# EUROPEAN PATENT APPLICATION

(11) **EP 2 842 482 A1**
(43) Date of publication of application: **04.03.2015**
(21) Application number: 12798586.9
(22) Date of filing: 16.05.2012
(51) Int. Cl.: A61B 5/021

(54) **BLOOD PRESSURE GAUGE WITH MICRO-ELECTRO-MECHANICAL SYSTEM (MEMS) MICROPHONE**

(30) Priority: 27.04.2012 CN 201210129817
(71) Applicant: Microlife Intellectual Property GmbH, Widnau 9443 St. Gallen (CH)
(72) Inventor: LIN, Chia-ming, Taipei City 114 (TW)
(74) Representative: Viering, Jentschura & Partner Patent- und Rechtsanwälte
(86) International application number: PCT/CN2012/075609
(87) International publication number: WO 2013/159410

(57) **Abstract**

A blood pressure gauge comprises a cuff, a gas pump, a pressure relief valve, a pressure sensor, a gas conduit, a MEMS microphone and a controller. The cuff has a gas bag. The gas pump is used for inflating and pressurizing the gas bag. The pressure relief valve is used for deflating and depressurizing the gas bag. The pressure sensor is used for detecting the pressure of the gas bag. The gas conduit connects the gas bag, the gas pump, the pressure relief valve and the pressure sensor to form a gas loop. The gas loop delivers gas among the gas bag, the gas pump, the pressure relief valve and the pressure sensor. The MEMS microphone is disposed at a specified location of the gas loop for detecting the Korotkoff sounds transmitted from the passing gas. The controller is used for monitoring and controlling the gas pump, the pressure relief valve, the pressure sensor and the MEMS microphone.

## Description

### BACKGROUND OF THE INVENTION

### 1. Field of the Invention

The invention relates to a blood pressure gauge having a MEMS (microelectromechanical systems) microphone for detecting Korotkoff sounds using the MEMS microphone.

### 2. Description of Related Art

When using an auscultation blood pressure gauge, a user wraps around his arm or wrist with a cuff containing a gas bag, and then inflates the gas bag of the cuff by pumping gas in. If the pressure of the pressurized gas bag is larger than a predetermined pressure for the standard systolic pressure of a normal person, the cuff highly pressurizes the artery of the wrist or arm so as to temporarily block the blood from going through the wrist or arm. Afterwards the user can open the pressure relief valve of the blood pressure gauge to gradually depressurize the gas bag. When the pressure of the deflated cuff is somewhat less than the systolic pressure of the artery, the artery starts to open, so some vibration sounds are resulted from swirls occurring in the blood of the artery where is pressurized by the cuff. Such vibration sounds are Korotkoff sounds. The pressure value obtained at the moment is called a systolic pressure value. Then, the pressure inside the cuff keeps dropping. When the Korotkoff sounds disappear, the pressure value obtained at the moment is called a diastolic pressure value.

Those conventional blood pressure gauges use condenser microphones (also called capacitor microphones) to detect Korotkoff sounds, as shown in the technical disclosure of the U.S. Patent publication No. 2008/0089527A1.

Because the SNR (signal-to-noise ratio) of the condenser microphone is directly proportional to its volume, the condenser microphone with a bigger volume is necessary for the blood pressure gauge to increase the sensitivity of Korotkoff sound detection. The condenser microphone currently used in the blood pressure gauge has a volume ranging from about 300-500 mm³. For this reason, such condenser microphone can just be disposed in a specified room with a volume of above 300-500 mm³. For example, it is disposed in a container larger than the diameter of a gas conduit.

Moreover, the condenser microphone receives sounds only from specific directions so that the receiving surface of the condenser microphone is aligned with only one direction perpendicular to the gas conduit when assembled. When the condenser microphone detects a Korotkoff sound, a voltage signal accordingly generated is transmitted to the voltage signal processing circuit of the blood pressure gauge. After the voltage signal needs to be calibrated by a circuit and converted from analog to digital, a corresponding blood pressure value is shown on a numeric display. Another room is further needed for housing the voltage signal processing circuit, and hence the volume of the blood pressure gauge cannot be effectively reduced. Consequently, there are some problems in the assembly of the blood pressure gauge and so on.

### SUMMARY OF THE INVENTION

The purpose of the invention is to solve the foregoing problems on the volume, the sound receiving orientation, the limitation of mounting location, and the room requirement of the voltage signal processing circuit of the conventional blood pressure gauge which uses the condenser microphone as a sound detecting device. Therefore, the present invention provides a sound detecting device of a blood pressure gauge to detect Korotkoff sounds. The foregoing sound detecting device comprises a gas loop and a MEMS microphone. The gas loop is used to deliver the gas. The MEMS microphone is used to detect Korotkoff sounds transmitted from passing gas. With regard to the foregoing sound detecting device of the blood pressure gauge, the MEMS microphone is disposed at a specified location of the gas loop.

The present invention further provides a blood pressure gauge comprising a cuff, a gas pump, a pressure relief valve, a pressure sensor, a gas conduit, a MEMS microphone and a controller. The cuff has a gas bag. The gas pump is used for inflating and pressurizing the gas bag. The pressure relief valve is used for deflating and depressurizing the gas bag. The pressure sensor is used for detecting the pressure of the gas bag. The gas conduit connects the gas bag, the gas pump, the pressure relief valve and the pressure sensor to form a gas loop. The gas loop delivers gas among the gas bag, the gas pump, the pressure relief valve and the pressure sensor. The MEMS microphone is disposed at a specified location of the gas loop for detecting the Korotkoff sounds transmitted from the passing gas. The controller is used for monitoring and controlling the gas pump, the pressure relief valve, the pressure sensor and the MEMS microphone.

### BRIEF DESCRIPTION OF THE DRAWINGS

FIG. 1 is a system diagram of the first embodiment of a blood pressure gauge according to the present invention;
FIG. 2 is a schematic diagram of the first embodiment of a sound detecting device according to the present invention;
FIG. 3 is a system diagram of the second embodiment of a blood pressure gauge according to the present invention;
FIG. 4 is a schematic diagram of the second embodiment of a sound detecting device according to the present invention;
FIG. 5 is a system diagram of the third embodiment of a blood pressure gauge according to the present invention; and
FIG. 6 is a schematic diagram of the third embodiment of a sound detecting device according to the present invention.

### DETAILED DESCRIPTION OF THE INVENTION

The blood pressure gauge of the present invention utilizes a MEMS microphone to detect sounds. Such a MEMS microphone has many advantages as follows: (1) its volume size is small; (2) its SNR is high; (3) its sensitivity is not affected by temperature variation; (4) its package body is small; (5) a correct audio signal can be obtained without passing a calibration circuit; (6) it is not easily interfered by external signals.

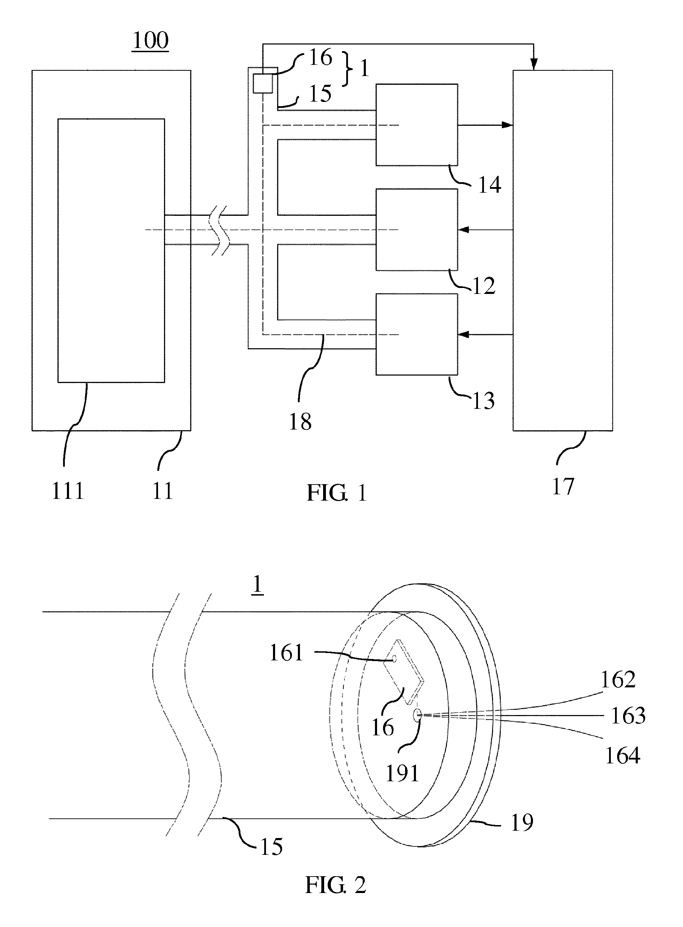

Referring to FIG. 1, it is a system diagram of the first embodiment of a blood pressure gauge according to the present invention. As shown in FIG. 1, a blood pressure gauge 100 comprises a cuff 11, a gas pump 12, a pressure relief valve 13, a pressure sensor 14, a gas conduit 15, a MEMS microphone 16 and a controller 17.

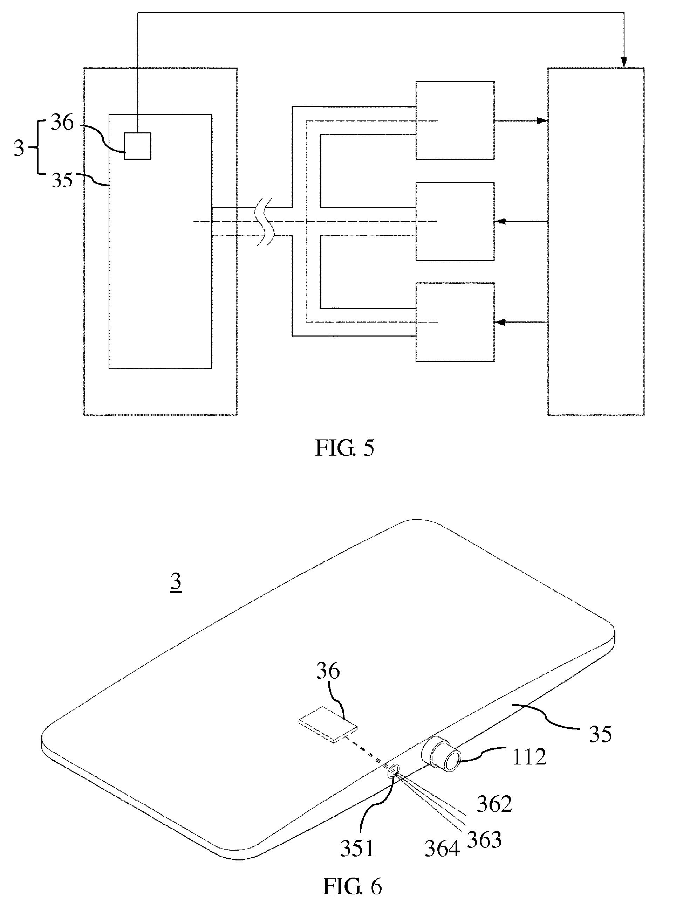

The cuff 11 has a gas bag 111. In the embodiment, the gas bag 111 is enclosed within the cuff 11, and has a gas communication portion (See the element labeled by numeral reference 112 as shown in FIG. 6) which can be connected to the gas conduit 15 (further described below) in some practical applications.

The gas pump 12 is used to inflate and pressurize the gas bag 111. In the embodiment, the gas pump 12 can pump any gas in the gas bag 111.

The pressure relief valve 13 is used to deflate and depressurize the gas bag 111.

The pressure sensor 14 is used to detect the pressure of the gas bag 111.

The gas conduit 15 connects the gas bag 111 of the cuff 11, the gas pump 12, the pressure relief valve 13 and the pressure sensor 14 to form a gas loop 18. The gas loop 18 delivers gas among the gas bag 111, the gas pump 12, the pressure relief valve 13, and pressure sensor 14.

The MEMS microphone 16 is a resistor microphone. It generally comprises a digital ASIC (application-specific integrated circuit) chip and a MEMS chip which are packaged in an SMT (surface mount technology) device. After the package is sealed by a lid, the SMT device has a sound receiving hole. With regard to the detailed structure of the MEMS microphone, it is not the point of the invention and thus will not be discussed here.

In this embodiment, the MEMS microphone 16 is disposed at a specified location in the gas loop 18 for detecting the Korotkoff sounds transmitted through the passing gas. Moreover, in the embodiment, the specified location is the position of the gas conduit 15. Therefore, the MEMS microphone 16 is disposed in the gas conduit 15 so that they serve as a sound detecting device 1 together.

The controller 17 is used to monitor and control the gas pump 12, the pressure relief valve 13, the pressure sensor 14, and the MEMS microphone 16.

Referring to FIG. 2, FIG. 2 is a schematic diagram of the first embodiment of a sound detecting device according to the present invention. As shown in FIG. 1 and FIG. 2, the sound detecting device 1 in the embodiment is obtained by disposing the MEMS microphone 16 in the gas conduit 15.

Concretely speaking, in the embodiment, the MEMS microphone 16 is mounted on an end cover 19 in a manner that the sound receiving hole 161 of the MEMS microphone 16 faces toward a direction opposite to the end cover 19 and is further disposed in the gas conduit 15 so as to receive the Korotkoff sounds transmitted from the gas. It is worthy to note that the end cover 19 has a through hole 191 and the MEMS microphone 16 has two power wires 162, 163 and a signal wire 164 which pass the through hole 191 and go along the gas conduit 15 to be connected with the controller 17 after the through hole 191 is filled with filler (not shown). In the embodiment, the material of the filler may be, but not limited to, silicone, resin, or polyurethane, or the combination thereof. The foregoing electrical connection between the controller 17 and the two power wires 162, 163 and the signal wire 164 of the MEMS microphone 16 belongs to the existing technique, so no further description is required here.

In other embodiments, after the MEMS microphone is disposed in the gas conduit, the sound receiving hole is aligned with the center of the gas conduit, and instead of the end cover, filler can be put into the gas conduit.

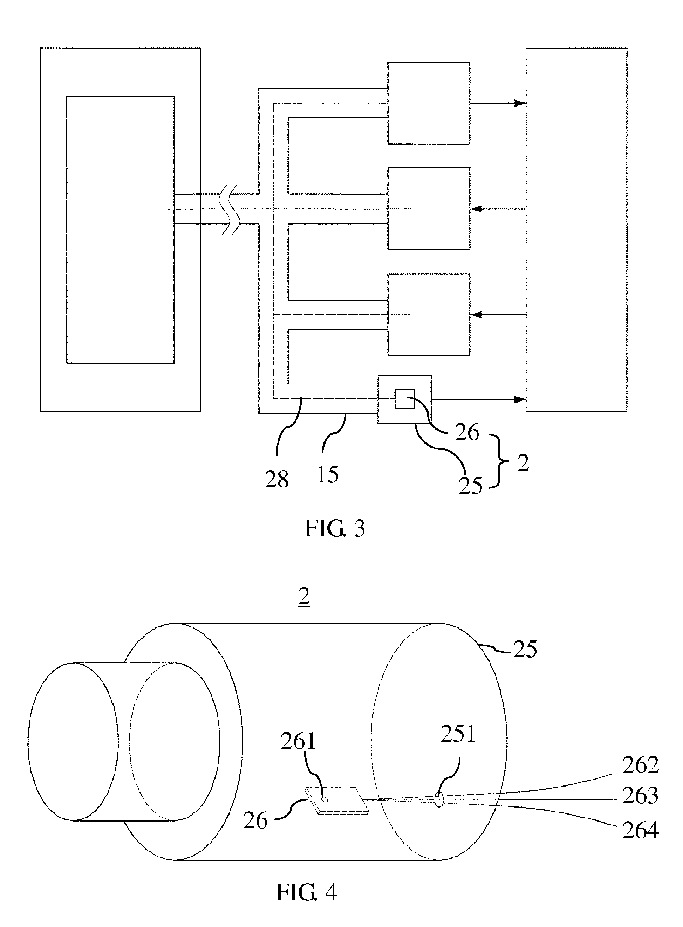

Referring to FIG. 3 and FIG. 4, FIG. 3 is a system diagram of the second embodiment of a blood pressure gauge according to the present invention, and FIG. 4 is a schematic diagram of the second embodiment of a sound detecting device according to the present invention. As shown in FIG. 3 and FIG. 4, the elements of the second embodiment are substantially the same as those of the first embodiment. The particular difference between them is that the gas conduit 15 of the present embodiment is further connected to a gas chamber 25 so as to form a gas loop 28. In addition, the specified location of the present embodiment is at the gas chamber 25, and the sound detecting device 2 is assembled by disposing the MEMS microphone 26 in the gas chamber 25.

Concretely speaking, the MEMS microphone 26 is disposed in the gas chamber 25. The gas chamber 25 has the through hole 251, and the sound receiving hole 261 of the MEMS microphone 26 faces toward a direction opposite the surfaces of the inner walls of the gas chamber 25. The MEMS microphone 26 has two power wires 262, 263 and a signal wire 264 which pass the through hole 251, and the through hole 251 is filled with filler (not shown).

Referring to FIG. 5 and FIG. 6, FIG. 5 is a system diagram of the third embodiment of a blood pressure gauge according to the present invention, and FIG. 6 is a schematic diagram of the third embodiment of a sound detecting device according to the present invention. As shown in FIG. 5 and FIG. 6, the elements of the third embodiment are substantially the same as those of the first embodiment. The particular difference between them is that the specified location of the present embodiment is on the inner surface of the gas bag 35. Moreover, the sound detecting device 3 is assembled by disposing the MEMS microphone 36 in the gas bag 35.

Concretely speaking, the MEMS microphone 36 is disposed on the inner surface of the gas bag 35. The gas bag 35 has the through hole 351, and the sound receiving hole (not shown) of the MEMS microphone 36 faces toward a direction opposite the inner surface of the gas bag 35 and is attached to the inner surface. The MEMS microphone 36 has two power wires 362, 363 and a signal wire 364 which pass the through hole 351, and the through hole 351 is filled with filler (not shown). A person skilled in the art would understand that the sound receiving hole of the MEMS microphone also can face the inner surface of the gas bag and is attached to the inner surface.

In other embodiments, the MEMS microphone also can be disposed on the outer surface of the gas bag. The sound receiving hole faces toward the outer surface of the gas bag, and is attached to the outer surface. Therefore, the sound receiving hole can receive the sounds generated by the vibrations caused from the gas bag.

The foregoing detailed description of the technology herein has been presented for purposes of illustration and description. It is not intended to limit the protective scope of the present invention. Many modifications and variations are possible in light of the above teaching. For example, the sequence of signal process or signal calibration can be changed. Furthermore, the system diagram of the blood pressure gauge can be modified by inserting or adding other function blocks, such as a screen for displaying a blood pressure value or the like, without affecting the technical content of the present invention.

## Claims

1. A sound detecting device of a blood pressure gauge for detecting Korotkoff sounds, comprising:
a gas loop for delivering gas; and
a MEMS microphone for detecting Korotkoff sounds transmitted from the gas passing therein;
wherein the MEMS microphone is disposed at a specified location of the gas loop.

2. The sound detecting device according to claim 1, wherein the specified location of the gas loop is at a gas chamber.

3. The sound detecting device according to claim 2, wherein the gas chamber has a through hole, the MEMS microphone has two power wires and a signal wire, and the power wires and the signal wire pass the through hole of the gas chamber and extend out from the gas chamber.

4. The sound detecting device according to claim 3, further comprising filler for filling in the through hole of the gas chamber.

5. The sound detecting device according to claim 1, wherein the specified location of the gas loop is at a gas conduit or at a gas bag.

6. The sound detecting device according to claim 5, wherein the MEMS microphone is disposed in the gas conduit.

7. The sound detecting device according to claim 5, wherein the MEMS microphone is disposed in the gas bag.

8. The sound detecting device according to claim 5, wherein the MEMS microphone is disposed outside the gas bag.

9. A blood pressure gauge, comprising:
a cuff having a gas bag;
a gas pump for inflating and pressurizing the gas bag;
a pressure relief valve for deflating and depressurizing the gas bag;
a pressure sensor for detecting a pressure of the gas bag;
a gas conduit connecting the gas bag, the gas pump, the pressure relief valve and the pressure sensor to form a gas loop, the gas loop delivering gas among the gas bag, the gas pump, the pressure relief valve and the pressure sensor;
a MEMS microphone disposed at a specified location of the gas loop for detecting Korotkoff sounds transmitted from the gas passing therein; and
a controller for monitoring and controlling the gas pump, the pressure relief valve, the pressure sensor and the MEMS microphone.

10. The blood pressure gauge according to claim 9, further comprising a gas chamber disposed in the gas loop.

11. The blood pressure gauge according to claim 10, wherein the specified location of the gas loop is at the gas chamber.

12. The blood pressure gauge according to claim 11, wherein the gas chamber has a through hole, the MEMS microphone has two power wires and a signal wire, and the power wires and the signal wire pass the through hole of the gas chamber to extend out of the gas chamber to be electrically connected to the controller.

13. The blood pressure gauge according to claim 12, further comprising filler filled in the through hole of the gas chamber.

14. The blood pressure gauge according to claim 9, wherein the specified location of the gas loop is at the gas conduit or the gas bag.

15. The blood pressure gauge according to claim 14, wherein the MEMS microphone is disposed in the gas conduit.

16. The blood pressure gauge according to claim 14, wherein the MEMS microphone is disposed in the gas bag.

17. The blood pressure gauge according to claim 14, wherein the MEMS microphone is disposed outside the gas bag and enclosed in the cuff.
